(19) Europäisches Patentamt
European Patent Office
Office européen des brevets

(11) **EP 4 127 716 B1**

(12) **EUROPEAN PATENT SPECIFICATION**

(45) Date of publication and mention of the grant of the patent:
**18.12.2024 Bulletin 2024/51**

(21) Application number: **21713420.4**

(22) Date of filing: **24.03.2021**

(51) International Patent Classification (IPC):
**G01N 33/543** (2006.01)   **G01N 33/533** (2006.01)
**G01N 33/558** (2006.01)

(52) Cooperative Patent Classification (CPC):
**G01N 33/543; G01N 33/533; G01N 33/54388**

(86) International application number:
**PCT/EP2021/057608**

(87) International publication number:
**WO 2021/191299 (30.09.2021 Gazette 2021/39)**

(54) **COMPOSITE PARTICLES FOR BIOLOGIC ASSAY**

KOMPOSITPARTIKEL FÜR EINEN BIOLOGISCHEN TEST

PARTICULES COMPOSITES POUR ANALYSE BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2020 EP 20165264**
        **12.01.2021 EP 21151045**

(43) Date of publication of application:
**08.02.2023 Bulletin 2023/06**

(73) Proprietor: **Nexdot**
**93230 Romainville (FR)**

(72) Inventors:
• **YAHIA AMMAR, Akram**
  **93230 Romainville (FR)**
• **KUNTZMANN, Alexis**
  **93230 Romainville (FR)**
• **D'AMICO, Michele**
  **93230 Romainville (FR)**
• **ALEZRA, Valérie**
  **93230 Romainville (FR)**
• **DUBERTRET, Benoît**
  **93230 Romainville (FR)**

(74) Representative: **Icosa**
**83 avenue Denfert-Rochereau**
**75014 Paris (FR)**

(56) References cited:
**EP-A1- 2 428 489      WO-A1-2018/220160**

**US-A1- 2009 294 742     US-A1- 2016 139 137**

• **MASAAKI KANAHARA ET AL: "Fabrication of gold nanoparticle-polymer composite particles with raspberry, core-shell and amorphous morphologies at room temperature via electrostatic interactions and diffusion", SOFT MATTER, vol. 10, no. 2, 1 January 2014 (2014-01-01), pages 275 - 280, XP055719534, ISSN: 1744-683X, DOI: 10.1039/C3SM52077C**
• **YASUFUMI MATSUMURA ET AL: "Metal (Au, Pt) Nanoparticle-Latex Nanocomposites as Probes for Immunochromatographic Test Strips with Enhanced Sensitivity", ACS APPLIED MATERIALS & INTERFACES, vol. 10, no. 38, 5 September 2018 (2018-09-05), US, pages 31977 - 31987, XP055719682, ISSN: 1944-8244, DOI: 10.1021/acsami.8b11745**
• **LI XUE ET AL: "Ultrasensitive lateral-flow assays based on quantum dot encapsulations with signal amplification", JOURNAL OF NANOPARTICLE RESEARCH, KLUWER ACADEMIC PUBLISHERS, DORDRECHT, NL, vol. 20, no. 5, 10 May 2018 (2018-05-10), pages 1 - 14, XP036524651, ISSN: 1388-0764, [retrieved on 20180510], DOI: 10.1007/S11051-018-4241-3**

- ZHOU CHANGHUA ET AL: "QDs embedded copolymer nanospheres prepared with a simple self-stable precipitation polymerization method for Listeria monocytogenes detection", OPTICAL MATERIALS, ELSEVIER SCIENCE PUBLISHERS B.V. AMSTERDAM, NL, vol. 99, 5 December 2019 (2019-12-05), XP086015116, ISSN: 0925-3467, [retrieved on 20191205], DOI: 10.1016/J.OPTMAT.2019.109582
- ZHANG JING ET AL: "Fluorescent microbeads for point-of-care testing: a review", MIKROCHIMICA ACTA, SPRINGER VERLAG, VIENNA, AT, vol. 186, no. 6, 17 May 2019 (2019-05-17), pages 1 - 21, XP036824986, ISSN: 0026-3672, [retrieved on 20190517], DOI: 10.1007/S00604-019-3449-Y
- CHAN LEE ET AL: "Feasibility of a point-of-care test based on quantum dots with a mobile phone reader for detection of antibody responses", PLOS NEGLECTED TROPICAL DISEASES, vol. 13, no. 10, 7 October 2019 (2019-10-07), pages e0007746, XP055719689, DOI: 10.1371/journal.pntd.0007746
- YAFEI HOU ET AL: "Smartphone-Based Dual-Modality Imaging System for Quantitative Detection of Color or Fluorescent Lateral Flow Immunochromatographic Strips", NANOSCALE RESEARCH LETTERS, SPRINGER, US, vol. 12, no. 1, 21 April 2017 (2017-04-21), pages 1 - 13, XP021244394, ISSN: 1931-7573, DOI: 10.1186/S11671-017-2078-9

**Description**

**FIELD OF INVENTION**

The present invention relates to composite particles for biologic assay.

**BACKGROUND OF INVENTION**

It is a perpetual challenge to respond to new and re-emerging infectious disease threats, which demands rapid response. Diagnostic tools are critical in the chain of response for patient care, resource allocation, disease containment, and public health surveillance. Point-of care diagnostics have gained attention for emergency situations because they are inexpensive, portable, operable by non-experts, and deliver results within minutes. For example, a biological fluid is added to an assay device comprising labels with specific binding properties. Upon binding of a label to a target biological material - virus, protein, nucleic acid, cell or metabolite - it becomes easy to detect presence of a target biological material in a sample. Detection may be done by eye or with simple optical devices using absorption or fluorescence of labels and a detector, for instance a camera in a smartphone.

In lateral flow immunoassays, a biological fluid is added on a strip, and it wicks through by capillary action. Two lines appear for a positive test, and one line for a negative test.

Sensitivity of such assays is limited by absorptive and emissive capabilities of labels. Traditional organic labels have numerous limitations when used to tag biological materials: absorption or emissive bands are usually large and cannot be easily designed to correspond to a predetermined light wavelength. Besides, their absorption or emission efficiency is low. Consequently, an organic label bound to a biological material gives a very low signal for an optical detector, lowering the sensitivity of the assay.

European patent application EP2428489 discloses quantum dot-embedded silica nanoparticle having plural quantum dots embedded within the silica nanoparticle, wherein the number of quantum dots existing in a concentric area within 10% of a radius from a center of the silica nanoparticle accounts for 10 to 70% of the number of total quantum dots embedded in the silica nanoparticle.

US patent application US2016/139137 discloses nanoparticle comprising a core comprising a first population of quantum dots (QDs) embedded in silica, and a shell comprising a second population of QDs embedded in silica.

In the publication "Fabrication of gold nanoparticle-polymer composite particles with raspberry, core-shell and amorphous morphologies at room temperature via electrostatic interactions and diffusion", by KANAHARA et al. Soft Matter, Vol. 10 (2), 2014, p. 275-280, polymer particles comprising gold nanoparticles are disclosed, wherein said nanoparticles are adsorbed on the surface of said polymer particles, are embedded in said polymer particles, or wherein the polymer particles comprising gold nanoparticles form a core/shell composite.

In the publication "Metal (Au, Pt) nanoparticle-latex nanocomposites as probes for immunochromatographic test strips with enhanced sensitivity", by MATSUMURA et al. ACS Applied Materials and Interfaces, Vol. 10 (38) 2018, p. 31977-31987, gold or platinum nanoparticles-latex nanocomposites are disclosed. Latex is poly(2-vinylpyridine) crosslinked with divynilbenzene. In the case of gold nanoparticles-latex nanocomposites, said Au nanoparticles have a part in the latex particle and a part outside the latex particle, *i.e.,* they are semi-embedded in latex. In the case of platinum nanoparticles-latex nanocomposites, said Pt nanoparticles are embedded and at the surface of latex particles.

In the publication "Ultrasensitive lateral-flow assays based on quantum dot encapsulations with signal amplification" by LI et al. Journal of nanoparticle research, Vol. 20 (5), 2018, p. 1-14, quantum dots in polymer particles are disclosed. The polymer is a modified tri-copolymer (poly(tert-butyl acrylate-coethyl acrylate-co-methacrylic acid).

In the publication "QDs embedded copolymer nanospheres prepared with a simple self-stable precipitation polymerization method for Lysteria monocytogenes detection", by ZHOU et al. Optical Materials, Vol. 99, 2019, p. 1-14, CdSe/ZnS quantum dots embedded in copolymer nanospheres are disclosed, wherein the copolymer is styrene-maleic anhydride copolymer.

In the publication "Fluorescent microbeads for point-of-care testing: a review" by ZHANG et al. Mikrochimica Acta, Vol. 186 (6), 2019, p. 1-21, polymer particles comprising quantum dots are disclosed. Three different structures are disclosed: quantum dots embedded in a polymer particle; polymer particle covered with a layer of quantum dots in a layer of silica; or silica particle encapsulating quantum dots, said silica particle being absorbed on the surface of a polymer particle.

International patent application WO2018220160 discloses composite particle comprising a plurality of nanoparticles encapsulated in an inorganic material.

US patent application US2009/294742 discloses nanocrystal-metal oxide-polymer composites. The composites comprise a number of nanocrystals within a metal oxide matrix, and an oligomer or polymer covalently bonded to organic reactive groups of the metal oxide matrix.

Thus, there is a continuing need in the assay art for labels with intense optical response for detection of very diluted biological material in a sample. Ideally, a single molecule of biological material of interest should be evidenced by one label.

In this disclosure, composite particles are used in order to improve sensitivity. When composite particles are large and dense enough in component with optical response, an intense optical signal is associated to a single binding between label and biological material: detection of very diluted biological material is improved.

## SUMMARY

The present invention relates to a composite particle, a detection method, and an assay test strip as defined in the appended claims.

## DEFINITIONS

In the present invention, the following terms have the following meanings:

- **"Mean size"** refers to a size of a population of particles, obtained by a mathematical mean of sizes of each individual particle of the population. Practically, the mean size may be determined by electronic microscopy: size of each particle visible in the microscopy is evaluated by fitting each particle with a circle whose diameter defines the size of the particle, then computing the mean of all individual sizes to obtain the mean size. Other methods, such as light scattering may be used to determine indirectly the mean size of the population of particles.
  Experimentally, particles are always obtained in the form of population of particles. By extension in this disclosure, the mean size of a particle is the mean size of the population of particles which has been synthesized. For the sake of clarity, a particle having a mean size between 50 nm and 1000 nm is a particle representative of a population of particles having a mean size between 50 nm and 1000 nm.

- **"Core/shell"** refers to a heterostructure in which a central nanoparticle: the core, is embedded by a layer of material disposed on the core: the shell. Two successive shells may be laid, yielding core/shell/shell heterostructure. Core and shell may have the same shape, for instance core is a nanosphere and shell is a layer of essentially constant thickness yielding a spherical core/shell nanoparticle.

- **"Nanometric size"** refers to a size of matter where at least one physical property is directly governed by size. For semi-conductive nanoparticles, nanometric size has to be defined with the average Bohr radius of an electron/hole pair in the material in which quantum effects appear. For semi-conductive materials disclosed here, quantum effects appear for size in at least one dimension of the object below 20 nm, preferably below 10 nm, more preferably below 5 nm. For conductive particles, nanometric size has to be defined from the resonance of oscillations of free electron gas density, which becomes relevant for optical measurements in the range from 380 nm to 3$\mu$m for size in at least one dimension of the object below 20 nm for conductive materials disclosed here.

- **"Nanoparticle"** refers to a particle having a size in at least one of its dimensions below 100 nm. For a nanosphere, diameter should be below 100 nm. For a nanoplate, thickness should be below 100 nm. For a nanorod, section size should be below 100 nm.

- **"Nanoplate"** refers to a two-dimensional shaped nanoparticle, wherein the smallest dimension of said nanoplate is smaller than the largest dimension of said nanoplate by a factor (aspect ratio) of at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5 or at least 10.

- **"Nanorod"** refers to a unidimensional shaped nanoparticle, wherein the smallest dimension of said nanorod is smaller than the largest dimension of said nanorod by a factor (aspect ratio) of at least 1.5, at least 2, at least 2.5, at least 3, at least 3.5, at least 4, at least 4.5, at least 5, at least 5.5, at least 6, at least 6.5, at least 7, at least 7.5, at least 8, at least 8.5, at least 9, at least 9.5 or at least 10

- **"Selective absorption"** refers to light absorption with an absorption band having a narrow FWHM (full width at half maximum). In the visible range - from 380 nm to 780 nm - a narrow FWHM is lower than 100 nm, preferably between 40 nm and 100 nm. In the near infra-red range - from 780 nm to 3 $\mu$m - a narrow FWHM is lower than 200 nm, preferably between 60 nm and 150 nm.

- **"Selective emission"** refers to light emission with an emission band having a narrow FWHM (full width at half maximum). In the visible range - from 380 nm to 780 nm - a narrow FWHM lower than 100 nm, preferably lower than 60 nm, ideally between 20 and 40 nm. In the near infra-red range - from 780 nm to 3 $\mu$m - a narrow FWHM is lower than 200 nm, preferably lower than 175 nm, ideally between 60 and 140 nm.

- **"Specific-binding component"** refers to a molecule that will selectively bind, through chemical or physical means to a detectable substance present in a sample, called herein "target analyte". "Selectively bind" means that the molecule binds preferentially to the target of interest or binds with greater affinity to the target than to other molecules. For example, an antibody will selectively bind to the antigen against which it was raised; a DNA molecule will bind to a substantially complementary sequence and not to unrelated sequences. The specific-binding component can comprise any molecule, or portion of any molecule, that is capable of being linked to a composite particle of the invention and that, when so linked, is capable of recognizing specifically a target analyte.

- **"Weight fraction"** defines the weight fraction of one material of a composite particle in said composite particle. For clarity, if 20 mg of nanoparticles A are dispersed in 80 mg of a matrix M, yielding 100 mg of composite particles, then weight fraction of nanoparticles A is 20%.

## DETAILED DESCRIPTION

### Composite particles

This disclosure relates to a composite particle comprising a matrix and nanoparticles dispersed in said matrix.
In this disclosure, nanoparticles have a selective optical property, *i.e.,* interaction with light in the ultra-violet range or in the visible range or in the infra-red range in a narrow range of wavelength.
In an embodiment, nanoparticles selectively absorb light.

### Nanoparticles with plasmonic resonance

Suitable nanoparticles are metallic nanoparticles whose absorption is governed by plasmonic effect, such as gold nanoparticles, silver nanoparticles or platinum nanoparticles. Other suitable nanoparticles are particles with an heterostructure - as defined below in section Quantum dot structure - with at least one sub-volume of metallic material. For instance, hollow metallic nanoparticles - such as gold, silver, platinum; cores/shell nanoparticles with a core of inorganic oxide - such as silica, alumina or zirconia - and a shell of metal - such as gold, silver, platinum; or cores/shell nanoparticles with a shell of inorganic oxide - such as silica, alumina or zirconia - and a core of metal - such as gold, silver, platinum - are suitable.
In an alternative embodiment, nanoparticles selectively emit light. Preferably, nanoparticles are inorganic.

### Luminescent rare earth-doped nanoparticles

Suitable nanoparticles are luminescent crystalline nanoparticles doped with rare earth elements. Crystalline matrix may be based on vanadate ions ($VO_4^{3-}$). Rare earth dopant may be selected from europium (Eu), dysprosium (Dy), samarium (Sm), praseodymium (Pr), neodymium (Nd), erbium (Er), ytterbium (Yb), cerium (Ce), holmium (Ho), terbium (Tb), thulium (Tm) and mixtures thereof.

### Fluorescent organo-metallic complexes

Suitable nanoparticles are fluorescent organo-metallic complexes, *i.e.,* a complex comprising a metallic element, preferably a transition metal, surrounded by an organic large molecule, preferably an aromatic molecule. These materials are normally solids and may be prepared at a nanometric size, then embedded in a matrix. Suitable organo-metallic complexes may be selected from the groups of porphyrines of transition metals, phthalocyanines of transition metals, chelates of transition metals, cryptates of transition metals, porphyrines of rare earths, phthalocyanines of rare earths, chelates of rare earths or cryptates of rare earths.

### Quantum dots

Other suitable nanoparticles are fluorescent nanoparticles known as quantum dots, which may have various composition, shape and structure.
As fluorescent particles first absorb light before re-emitting light, fluorescent particles are also absorbing particles.

*Quantum dots composition*

In one embodiment, the quantum dots comprise a material of formula $M_xQ_yE_zA_w$ (I), in which M is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; Q is selected from the group consisting of Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, Cs or a mixture thereof; E is selected from the group consisting of O, S, Se, Te, C, N, P, As, Sb, F, Cl, Br, I, or a mixture thereof; and A is selected from the group consisting of O, S, Se, Te, C, N, P, As, Sb, F, Cl, Br, I, or a mixture thereof. x, y, z and w are independently a decimal number from 0 to 5; x, y, z and w are not simultaneously equal to 0; x and y are not simultaneously equal to 0; z and w may not be simultaneously equal to 0.

In particular, quantum dots may comprise a material of formula $M_xE_y$, in which M is Zn, Cd, Hg, Cu, Ag, Al, Ga, In, Si, Ge, Pb, Sb or a mixture thereof; and E is O, S, Se, Te, N, P, As or a mixture thereof. x and y are independently a decimal number from 0 to 5, with the proviso that x and y are not 0 at the same time.

In a specific embodiment, quantum dots comprise a material selected from the group consisting of CdS, CdSe, CdTe, ZnS, ZnSe, ZnTe, HgS, HgSe, HgTe, HgO, GeS, GeSe, GeTe, SnS, SnSe, SnTe, PbS, PbSe, PbTe, $GeS_2$, $GeSe_2$, $SnS_2$, $SnSe_2$, $CuInS_2$, $CuInSe_2$, $AgInS_2$, $AgInSe_2$, CuS, $Cu_2S$, $Ag_2S$, $Ag_2Se$, $Ag_2Te$, FeS, $FeS_2$, InP, $Cd_3P_2$, $Zn_3P_2$, CdO, ZnO, FeO, $Fe_2O_3$, $Fe_3O4$, $Al_2O_3$, $TiO_2$, MgO, MgS, MgSe, MgTe, AlN, AlP, AlAs, AlSb, GaN, GaP, GaAs, GaSb, InN, InP, InAs, InSb, TlN, TlP, TlAs, TlSb, $MoS_2$, PdS, Pd4S, $WS_2$, $CsPbCl_3$, $PbBr_3$, $CsPbBr_3$, $CH_3NH_3PbI_3$, $CH_3NH_3PbCl_3$, $CH_3NH_3PbBr_3$, $CsPbI_3$, $FAPbBr_3$ (where FA stands for formamidinium), or a mixture thereof.

In a specific embodiment, quantum dots are carbon dots.

In one embodiment, quantum dots are doped with at least one transition metal such as, for example, Sc, Y, Ti, Zr, Hf, Rf, V, Nb, Ta, Dd, Cr, Mo, W, Sg, Mn, Tc, Re, Bh, Fe, Ru, Os, Hs, Co, Rh, Ir, Ni, Pd, Pt, Cu, Ag or Au. Preferably, quantum dots are doped with Mn or Ag.

*Quantum dots shape*

In one embodiment, quantum dots may have different shapes, provided that they present a nanometric size leading to quantum confinement in the nanoparticle.

Quantum dots may have nanometric sizes in three dimensions, allowing quantum confinement in all three spatial dimensions. Such quantum dots are for instance nanocubes or nanospheres.

Quantum dots may have a nanometric sizes in two dimensions, the third dimension being larger: quantum confinement is in two spatial dimensions. Such quantum dots are for instance nanorods, nanowires or nanorings.

Quantum dots may have a nanometric size in one dimension, the other dimensions being larger: quantum confinement is in one spatial dimension only. Such quantum dots are for instance nanoplates, nanosheets, nanoribbons or nanodisks. Nanoplates are especially interesting in this disclosure because cross section - *i.e.,* efficiency to capture a photon of incident light on the quantum dot - is ten times higher than a nanosphere having the same composition and structure. This higher cross section improves significantly sensitivity of assays.

The exact shape of quantum dots defines confinement properties; then electronic and optical properties.

*Quantum dots structure*

In an embodiment, quantum dots are homostructures. By homostructure, it is meant that the quantum dot is homogenous and has the same local composition in all its volume.

In an alternative embodiment, quantum dots are heterostructures. By heterostructure, it is meant that the quantum dot is comprised of several sub-volumes, each sub-volume having a different composition from neighbouring sub-volumes. In a particular embodiment, all sub-volumes have a composition defined by formula (I) disclosed above, with different parameters, *i.e.,* elemental composition and stoichiometry.

Example of heterostructure are core/shell nanoparticles, the core having any shape disclosed above. A shell is a layer covering totally or partially the core. A particular example of core/shell heterostructure is a multi-layered structure comprising a core and several successive shells. For convenience, these multi-layered heterostructures are named core/shell hereafter. Core and shell may have the same shape - sphere in sphere for example - or not - sphere in plate for instance.

Another example of heterostructure are core/crown nanoparticles, the core having any shape disclosed above. A crown is a band of material disposed on the periphery of the core. This heterostructure is particularly useful with cores being nanoplates and crown disposed on the edges of the nanoplate.

In a configuration, nanoparticles are selected from $CdSe_xS_{(1-x)}/CdS/ZnS$, $CdSe_xS_{(1-x)}/Cd_yZn_{(1-y)}S$, Cd-

$Se_xS_{(1-x)}$/ZnS, $CdSe_xS_{(1-x)}$/$Cd_yZn_{(1-y)}$S/ZnS, $CdSe_xS_{(1-x)}$/CdS, CdSe/CdS/ZnS, CdSe/CdS, CdSe/$Cd_yZn_{(1-y)}$S, CdSe/$Cd_yZn_{(1-y)}$S/ZnS, $CdSe_xS_{(1-x)}$/CdS/ZnSe, $CdSe_xS_{(1-x)}$/$Cd_yZn_{(1-y)}$Se, $CdSe_xS_{(1-x)}$/ZnSe, $CdSe_xS_{(1-x)}$/$Cd_yZn_{(1-y)}$Se/ZnSe, $CdSe_xS_{(1-x)}$/$Cd_yZn_{(1-y)}$Se/ZnS, CdSe/CdS/ZnSe, CdSe/$Cd_yZn_{(1-y)}$Se, CdSe/$Cd_yZn_{(1-y)}$Se/ZnSe, CdSe/$Cd_yZn_{(1-y)}$Se/ZnS, $CdSe_xS_{(1-x)}$/CdS/$ZnSe_yS_{(1-y)}$, $CdSe_xS_{(1-x)}$/$Cd_yZn_{(1-y)}$S, $CdSe_xS_{(1-x)}$/$ZnSe_yS_{(1-y)}$, $CdSe_xS_{(1-x)}$/$Cd_yZn_{(1-y)}$S/$ZnSe_zS_{(1-z)}$, $CdSe_xS_{(1-x)}$/CdS, CdSe/CdS/$ZnSe_yS_{(1-y)}$, CdSe/CdS, CdSe/$Cd_yZn_{(1-y)}$S, CdSe/$Cd_yZn_{(1-y)}$S/ $ZnSe_zS_{(1-z)}$, $CdSe_xS_{(1-x)}$/CdS/$ZnSe_yS_{(1-y)}$, $CdSe_xS_{(1-x)}$/$Cd_yZn_{(1-y)}$Se, $CdSe_xS_{(1-x)}$/$ZnSe_yS_{(1-y)}$, $CdSe_xS_{(1-x)}$/$Cd_yZn_{(1-y)}$Se/$ZnSe_zS_{(1-z)}$, $CdSe_xS_{(1-x)}$/$Cd_yZn_{(1-y)}$Se/$ZnSe_zS_{(1-z)}$, CdSe/$Cd_yZn_{(1-y)}$Se, CdSe/$Cd_yZn_{(1-y)}$Se/$ZnSe_zS_{(1-z)}$, CdSe/$Cd_yZn_{(1-y)}$Se/$ZnSe_zS_{(1-z)}$ where x, y and z are rational numbers between 0 (excluded) and 1 (excluded), and emit light by fluorescence. Emitted light is typically a band centered on a wavelength in the visible range from 380 nm to 780 nm.

Suitable nanoparticles emitting red light at 630 nm with FWHM of 25 nm are core/shell/shell nanoplatelets of $CdSe_{0.45}S_{0.55}$/$Cd_{0.30}Zn_{0.70}$S/ZnS, with a core of thickness 1.2 nm and a lateral dimension, i.e., length or width, greater than 8 nm and shells of thicknesses 2.5 nm and 2 nm. Other suitable nanoparticles emitting red light at 653 nm with FWHM of 25 nm are core/shell/shell nanoplatelets of $CdSe_{0.72}S_{0.28}$/CdS/ZnS, with a core of thickness 1.2 nm and a lateral dimension, i.e., length or width, greater than 8 nm and shells of thicknesses 3.5 nm and 0.5 nm. Other suitable nanoparticles emitting red light at 652 nm with FWHM of 20 nm are core/shell/shell nanospheres of CdSe/CdS/ZnS, with a core of diameter 4.5 nm and shell thicknesses of 1.5 nm and 1 nm. Other suitable nanoparticles emitting red light at 630 nm with FWHM of 45 nm are core/shell/shell nanospheres of InP/$ZnSe_{0.50}S_{0.50}$/ZnS, with a core of diameter 3.5 nm and shell thicknesses of 2 nm and 1 nm. Other suitable nanoparticles emitting red light at 630 nm with FWHM of 40 nm are core/shell nanospheres of InP/ZnS, with a core of diameter 3.5 nm and shell thicknesses of 2 nm. Other suitable nanoparticles emitting red light at 635 nm with FWHM of 40 nm are core/shell nanospheres of InP/ZnSe, with a core of diameter 3.5 nm and shell thicknesses of 2 nm. Other suitable nanoparticles emitting red light at 625 nm with FWHM of 40 nm are core/shell nanoparticles of $CuInSe_2$/ZnS. Other suitable nanoparticles emitting red light at 625 nm with FWHM of 40 nm are core/shell nanoparticles of $CuInS_2$/ZnS. Other suitable nanoparticles emitting red light at 625 nm with FWHM of 40 nm are carbon dots. Other suitable nanoparticles emitting red light at 635 nm with FWHM of 35 nm are core/shell nanoparticles of CdSe/CdS doped with Mn. Other suitable nanoparticles emitting red light at 645 nm with FWHM of 35 nm are core/shell nanoparticles of CdSe/CdS doped with Ag.

Suitable nanoparticles emitting green light at 530 nm with FWHM of 30 nm are core/shell/shell nanoplatelets of $CdSe_{0.32}S_{0.68}$/ZnS/$Cd_{0.15}Zn_{0.85}$S, with a core of thickness 1.2 nm and a lateral dimension, i.e., length or width, greater than 10 nm and shells of thicknesses 1 nm and 2.5 nm. Other suitable nanoparticles emitting green light at 540 nm with FWHM of 37 nm are core/shell nanospheres of $CdSe_{0.10}S_{0.90}$/ZnS, with a core of diameter 4 nm and shell thickness of 1 nm.

Suitable nanoparticles emitting blue light at 450 nm with FWHM of 30 nm are core/shell nanoplatelets of CdS/ZnS, with a core of thickness 0.9 nm and a lateral dimension, i.e., length or width, greater than 15 nm and a shell of thickness 1 nm.

In this disclosure, nanoparticles have a size in at least one of its dimensions shorter than 20 nm, preferably a nanometric size. For spherical nanoparticles, diameter should be less than 20 nm. For a nanoplate, thickness should be less than 20 nm. For a nanorod, section size should be less than 20 nm. In a specific embodiment, nanoparticles have a size in at least one of its dimensions shorter than 10 nm, preferably shorter than 5 nm.

In this disclosure, the weight fraction of nanoparticles in composite particle is greater than 0.5%. In a preferred embodiment, weight fraction of nanoparticles in composite particle is greater than 1%, more preferably greater than 2%, even preferably greater than 5%. Indeed, optical property of composite particle is the sum of optical properties of nanoparticles comprised in said composite particle: a high weight fraction corresponds to intense optical response and improved sensitivity. It has been surprisingly observed that a weight fraction of nanoparticles in composite particle less than 0.5% do not provide a sufficient optical response: in other words, they do not contain enough nanoparticles to improve sensitivity of assay.

In this disclosure, the weight fraction of nanoparticles in composite particle is less than 50%. Indeed, mechanical stability of composite particles is brought by matrix and composite particles in which weight fraction of matrix is less than 50% are fragile: they break easily during operations, yielding isolated nanoparticles and lowering sensitivity of assay. Besides, if weight fraction of matrix is too low, nanoparticles may not be completely embedded in the matrix, hence being present at the surface of the composite particle and finally perturbate the correct chemical/physical formation of composite particles and their surface response to biological functionalization. In an embodiment, the weight fraction of nanoparticles in composite particle is less than 40%, preferably less than 30%.

According to a specific embodiment, weight fraction of nanoparticles in composite particle may be in one of the following range: 0.5%-40%; 0.5%-30%; 0.5%-25%; 0.5%-20%; 0.5%-15%; 0.5%-12%; 0.5%-10%; 1%-50%; 1%-40%; 1%-30%; 1%-25%; 1%-20%; 1%-15%; 1%-12%; 1%-10%; 2%-50%; 2%-40%; 2%-30%; 2%-25%; 2%-20%; 2%-15%; 2%-12%; 2%-10%; 3%-50%; 3%-40%; 3%-30%; 3%-25%; 3%-20%; 3%-15%; 3%-12%; 3%-10%; 4%-50%; 4%-40%; 4%-30%; 4%-25%; 4%-20%; 4%-15%; 4%-12%; 4%-10%; 5%-50%; 5%-40%; 5%-30%; 5%-25%; 5%-20%; 5%-15%;

5%-12% or 5%-10%.

More specifically, weight fraction of nanoparticles having a nanoplate shape in composite particle may be in one of the following range: 0.5%-20%; 0.5%-15%; 0.5%-12%; 0.5%-10%; 1%-50%; 1%-20%; 1%-15%; 1%-12%; 1%-10%; 2%-20%; 2%-15%; 2%-12%; 2%-10%; 3%-20%; 3%-15%; 3%-12%; 3%-10%; 4%-20%; 4%-15%; 4%-12%; 4%-10%; 5%-20%; 5%-15%; 5%-12% or 5%-10%. The lower weight fraction of nanoparticles having a nanoplate shape in composite particle is balanced by the higher absorption cross section of nanoplates to yield a very efficient sensitivity in assays.

In this disclosure, composite particle is functionalized with a specific-binding component, so that composite particle may bind to a biological material. Functionalization may be done by grafting or absorption of the specific-binding component on the surface of the composite particle. Specific-binding component includes but is not limited to: antigens, steroids, vitamins, drugs, haptens, metabolites, toxins, environmental pollutants, amino acids, peptides, proteins, antibodies, polysaccharides, nucleotides, nucleosides, oligonucleotides, psoralens, hormones, nucleic acids, nucleic acid polymers, carbohydrates, lipids, phospholipids, lipoproteins, lipopolysaccharides, liposomes, lipophilic polymers, synthetic polymers, polymeric microparticles, biological cells, virus and combinations thereof. Preferred peptides include, but are not limited to: neuropeptides, cytokines, toxins, protease substrates, and protein kinase substrates. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins and growth factors. Preferred nucleic acid polymers are single- or multi-stranded, natural or synthetic DNA or RNA oligonucleotides, or DNA/RNA hybrids, or incorporating an unusual linker such as morpholine derivatized phosphides, or peptide nucleic acids such as N-(2-aminoethyl)glycine units, where the nucleic acid contains fewer than 50 nucleotides, more typically fewer than 25 nucleotides.

In this disclosure, the composite particle has a mean size greater than 50 nm and less than 1000 nm, preferably greater than 150 nm, more preferably greater than 250 nm.

It has been surprisingly observed that composite particles with a size less than 50 nm and weight fraction of nanoparticles in composite particle greater than 0.5% do not provide a sufficient optical response: in other words, they do not contain enough nanoparticles to improve sensitivity of assay. Indeed, composite particle need to be larger than nanoparticles so as to embed several nanoparticles and provide with improvement of sensitivity of assay. In other words, small composite particles cannot have a high load of nanoparticles due to geometric constraints, whereas big composite particles can have a higher load of nanoparticles leading to an enhanced fluorescence intensity, thus to an improved sensitivity of the assay.

In addition, composite particles with a size greater than 1000 nm are not suitable for two reasons. First, dispersion in aqueous medium is limited by sedimentation effect. Second, strength of binding with biological material becomes much less than hydrodynamic forces exerted on the composite particles. Both effects limit the ability of composite particle to bind with the biological material, hence limits sensitivity of assay.

In some conditions of biologic assay, depending on the dispersion of composite particles and the medium in which assay is performed, especially if medium is a porous material, composite particles have preferably a size less than 800 nm, preferably less than 600 nm, more preferably less than 400 nm.

Without being bound by theory, it is believed that composite particles functionalized with specific-binding component have a high ratio of optical response per specific binding sites. Indeed, improving sensitivity of assay requires to associate a single target analyte with a label having intense optical response. Composite particles of this disclosure show a balance between availability for assay - composite particles are small enough to offer a large contact surface and disperse in liquid medium - and sensitivity in optical measurements - with nanoparticles having high cross section in their interaction with light and/or high fluorescence yield.

Composite particles may be characterized by their optical capacity, defined here as the product of the cross section of said composite particle multiplied by the yield of optical effect, *i.e.,* the percentage of photons arriving on an absorbing composite particle and being actually absorbed or the percentage of photons arriving on an emitting composite particle and actually leading to a photon emission by luminescence.

Besides, composite particles may be defined by their biological capacity, defined here as the number of specific-binding component per unit surface of composite particles.

According to an embodiment, the composite particle comprises a first fraction NP1 of nanoparticles that selectively absorbs or selectively emits light; and a second fraction NP2 of nanoparticles that selectively absorbs or selectively emits light differently from NP1. With this configuration, detection of the target analyte is associated with a combination of two optical responses: two bands of absorption or two bands of emission or one band of absorption and one band of emission. Besides, the amounts of fractions NP1 and NP2 define the relative intensity of optical measurements. More than two fractions, for instance, three fractions or four fractions may be comprised in composite particles. One is then able to design a library of composite particles having different optical responses and to associate each composite particle of the library with a specific-binding component. Finally, an assay may be conducted with a mixture of composite particles, each composite particle being able to bind with a target analyte and each composite particle being identified by optical measurement. Multiple assay is thus conducted on a single sample. As sensitivity of assay is improved with composite particle disclosed above, decreasing the concentration of each type of composite particle is not an issue and multi-assay is much more reliable.

According to the invention, the matrix of the composite particle is inorganic, and comprises $SiO_2$, $Al_2O_3$, $ZrO_2$, $HfO_2$, or a mixture thereof such as, for example, $Si_{1-x}Zr_xO_2$, $Al_{2-2x}Zr_{2x}O_{(3+x)}$, $Hf_{1-x}Zr_xO_2$, x being a rational number between 0 (excluded) and 1 (excluded). These mixed oxides can also be noted (100-y)% by weight of $SiO_2$ / y% by weight of $ZrO_2$, (100-y)% by weight of $Al_2O_3$ / y% by weight of $ZrO_2$, or (100-y)% by weight of $HfO_2$ / y% by weight of $ZrO_2$, y being a rational number between 0 (excluded) and 100 (excluded). In such mixtures, the molar fraction of zirconium is preferably ranging from 0 to 20%, preferably from 0 to 10%. Using the oxides or mixtures of oxides as listed here has tremendous advantages: it prevents $O_2$ diffusion inside the particle; it leads to denser particles especially compared to silica alone.

According to the invention, the matrix of the composite particle comprises less than 90% by weight of $SiO_2$ (silica), more preferably less than 80% by weight of $SiO_2$, even more preferably less than 75% by weight of $SiO_2$, based on the weight of said matrix. Using pure $SiO_2$ as a matrix encapsulating nanoparticles requires said nanoparticles to be dispersible in ethanol. However, that is rarely the case with quantum dots which are typically soluble in solvents such as heptane, thus a ligand exchange is required to disperse said quantum dots from heptane to ethanol, this step being detrimental to the fluorescence properties of the quantum dots. Using a matrix comprising at least less than 90% of $SiO_2$ eliminates this step. Also, the resulting composite particle will be denser than with pure $SiO_2$ as the matrix, thereby preventing $O_2$ diffusion inside the particle.

Herein, the percentage by weight (also called mass fraction) of silica in the matrix refers to the equivalent mass of silica in said matrix, *i.e.,* the mass of silica in the matrix divided by the total mass of said matrix. For example, concerning a matrix comprising 90 mol% of $SiO_2$ and 10 mol% $ZrO_2$, the mass fraction of $SiO_2$ in said matrix can be calculated as follows:

$$w(SiO_2) = \frac{0.90*M(SiO2)}{0.90*M(SiO2)+0.10M(ZrO2)} = 0.81$$

wherein w is a molar fraction, and M is a molar mass.

Said mass fraction is typically determined by energy-dispersive X-ray spectroscopy (EDX).

In a specific configuration of this embodiment, the matrix comprises less than 1% by weight of $SiO_2$ based on the total weight of the matrix.

**Method of detection of an analyte**

The invention also relates to a method of detection of a target analyte.

In a first step, a sample is provided. Sample may be related to human health and selected from nasopharyngeal wash, blood, plasma, cell-free plasma, buffy coat, saliva, urine, stool, sputum, mucous, wound swab, tissue biopsy, milk, a fluid aspirate, a swab (e.g., a nasopharyngeal swab), and/or tissue. Sample may be related to environment and selected from water, food extract (vegetal or animal), soil. The sample is likely to contain a target analyte whose detection is desirable.

In a second step, sample and composite particles disclosed above are put in contact, usually in liquid form, *i.e.,* a solution. The specific-binding component of composite particles is selected so as to bind the target analyte. Contact of sample and composite particles may last from a few seconds to hours. Contact of sample and composite particles may be operated in various devices suitable to handle a solution, including microfluidic devices. Simple vials are suitable, as well as more elaborated devices allowing for migration of the sample through a medium in which composite particles are initially located and known as chromatographic assays. Other setups in which composite particles are injected in cells or tissues are also suitable. In the second step, composite particles bind with target analyte.

In a third step, composite particles bound with target analyte are separated from composite particles not bound with said target analyte. By separated, it is meant that the population of composite particles bound with target analyte and the population of composite particle not bound with target analyte are split in two parts, allowing to proceed with the method on one population of composite particles only. Separation may be operated in various ways. Composite particles bound with target analyte may be adsorbed on a surface where target analyte binds chemically or binds with another specific-binding component, yielding a "sandwich" configuration: target analyte is bound by two specific-binding agents, one to attach the target analyte to a surface and one to attach a label to the target analyte. Alternatively, composite particles bound with target analyte may be aggregated with a another specific-binding component: aggregates may be then separated by centrifugation or sedimentation. In another approach, composite particles may be sorted by cytometric flow: each particle (bound or not bound) flows individually through a channel where its optical properties are measured and upon result of optical measure, particles are flown in a reservoir for bound composite particles and another reservoir for not bound composite particles. In another approach, washing steps may be done to remove not bound composite particles from sample while bound composite particles remain attached to the sample: this would be particularly suitable for assay associated with imaging in cells or tissues.

Last, in a fourth step, optical measurement is run with one population of composite particle. If composite particles bound with the target analyte are measured, this is a direct measure of presence of analyte. In some embodiments, measurement allows for a quantitative estimate of analyte concentration in sample. If composite particles not bound with the target analyte are measured, this is an indirect measure: the difference of optical signal from composite particles before contact with sample and after contact with sample is related to binding with target analyte.

Optical measurement may be in Ultra-violet (280 nm - 380 nm), Visible (380 nm - 780 nm) or Near Infra-red (780 nm - 3 $\mu$m) range of light.

In the fourth step, optical measurement may be a measure of absorption of the population of composite particles. Alternatively, optical measurement may be a measure of emission of the population of composite particles, especially a measure of luminescence. When composite particles comprise at least two fractions NP1 and NP2 of nanoparticles, optical measurement may be a combination absorption and emission measurements.

In an embodiment, sample is put in contact with several composite particles of different types. By different types, it is meant composite particles comprising different nanoparticles or different mixture of nanoparticles. In other words, composite particles of different types have different optical responses, by wavelength of absorption/emission or by spectrum comprising several bands of absorption/emission of specific central wavelength and relative intensity. This embodiment is particularly adapted for multiple testing, each type of composite particle being functionalized with a type of specific-binding component.

In an embodiment, second and third steps disclosed above are performed on a strip, preferably a strip of porous material. This configuration allows for chromatographic measurement. Initially, composite particles are disposed on an area $A_{cp}$ of the strip. Then sample is deposited on the strip on an area $A_{sa}$, on the side of $A_{cp}$. Sample is then obliged to flow through $A_{cp}$, for instance by capillary forces in the strip: this is the contact step. Another area of the strip $A_{test}$ may be grafted with another specific-binding agent of the target analyte. By forcing sample to flow through $A_{test}$ after flowing through $A_{cp}$, composite particles bound to target analyte attach to $A_{test}$ where they will be optically detected later.

In an embodiment, optical measure is made with a portable device, preferably a mobile phone or a smartphone. Indeed, mobile phones and smartphones usually comprise light sources in Ultra-violet, Visible and/or Near Infra-red range of light and camera suitable to measure light in Visible and/or Near Infra-red range of light. All optical measurements may be performed with such a smartphone: absorption or luminescence.

**Assay test strip**

The invention also relates to an assay test strip.

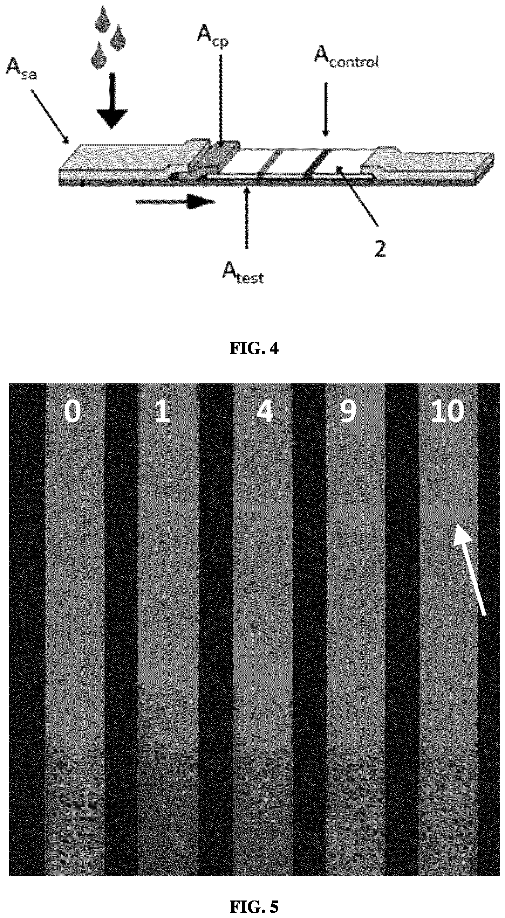

As illustrated in Figure 4, the assay test strip comprises a porous substrate (2), a sample receiving zone $A_{sa}$ and composite particle as disclosed above that specifically binds a target analyte. Composite particles are disposed on an area $A_{cp}$ of the strip.

In addition, the strip comprises a detection zone. A first immobilized reagent that specifically binds said target analyte is disposed in area $A_{test}$, in the detection zone, preferably under the form of a line.

Last, a second immobilized reagent that specifically binds with specific-binding component of composite particles is disposed in area $A_{control}$, in the detection zone, preferably under the form of a line. The second immobilized reagent does not bind with a composite particle bound to the target analyte.

In operation, sample deposited on $A_{sa}$ is forced by capillary forces to flow through $A_{cp}$ where target analyte binds to composite particles. Then composite particles are transported by the flow towards the detection zone. If composite particle is bound to target analyte, then analyte will bind to the first immobilized reagent, thus attaching composite particle on $A_{test}$, preferably in the form of a line. If composite particle is not bound to target analyte, then specific-binding component is available for binding with the second immobilized reagent, thus attaching composite particle, preferably in the form of a line. Upon optical measurement, absorption or emission of light in $A_{test}$ demonstrates that target analyte was present in the sample. Besides, absorption or emission of light in $A_{control}$ demonstrates that composite particles have been transported correctly by the flow and that assay operation is valid.

In a variant, composite particles are functionalized with one specific-binding components able to bind on two sites. One binding site is specific for target analyte. The other binding site is specific for the second immobilized reagent. In this variant, composite particle bound to target analyte is available for control: this is particularly useful if concentration of target analyte is very high, in excess as compared to composite particles.

In another variant, composite particles are functionalized with two specific-binding components, one being designed to bind with analyte then bind on $A_{test}$, the second being designed to bound to $A_{control}$. In another variant, composite particles are functionalized either with one specific-binding component being designed to bind with analyte

then bind on $A_{test}$, or with one second specific-binding component being designed to bound to $A_{control}$ and a mixture of both types of composite particles is used.

**BRIEF DESCRIPTION OF THE DRAWINGS**

**Figure 1** describes assay device and protocol used in example 1-a.

**Figure 2** is a series of photograph of assay results from example 1.

**Figure 3** is a series of fluorescence microscopy pictures of results from example 1-b.

**Figure 4** describes an assay test strip.

**Figure 5** is a series of photographs of assay results from example 2.

**Figure 6** is a series of photographs of assay results from example 3.

**EXAMPLES**

Table 1 below lists all composite particles used in experiments, as well as test results.
NS stands for nanosphere. Dimensions are diameter of the sphere or diameter of the core and thickness of the successive shells.
NPL stands for nanoplate. Dimensions are length, width and thickness. Thickness is always the smallest dimension in nanoplates.

(1) stands for intensity of light measured in a fluorescence test (arbitrary unit) using an inverted fluorescence microscope LEICA DMi8, with HXP 120 lamp (Gain = 1, water objective 63x, excitation with a UV-violet band of 20 nm centered at 390 nm), with an integration of signal over a time of n milliseconds (n varies from 1 to 500 ms).

(2) stands for intensity of light absorption.

Composite particles (#4 to #11) are obtained by a spray process: 500 $\mu$L of $CdSe_{0.72}S_{0.28}$/CdS/ZnS nanoplates suspended in water (10 mg/mL) were mixed with 20 mL of water, tetraethoxysilane (TEOS) and ammonia, then loaded on a spray-drying set-up. The liquid was sprayed towards a tube furnace heated at a temperature ranging from the boiling point of the solvent to 1000 °C, with a nitrogen flow. The composite particles $CdSe_{0.72}S_{0.28}$/CdS/ZnS in $SiO_2$ matrix were collected at the surface of a filter.
Composite particles (#12 to #24) are obtained by a simultaneous spray process: 500 $\mu$L of nanoplates or nanospheres suspended in heptane (10 mg/mL) were mixed with aluminum tri-sec butoxide and 20 mL of cyclohexane, then loaded on a spray-drying set-up. On another side, an aqueous solution was prepared and loaded the same spray-drying set-up, but at a different location than the first heptane solution. The two liquids were sprayed simultaneously towards a tube furnace heated at a temperature ranging from the boiling point of the solvent to 1000 °C, with a nitrogen flow. The composite particles comprising nanoplates or nanospheres in $Al_2O_3$ were collected at the surface of a filter.
Composite particles (#25) are obtained by a spray process: 500 $\mu$L of $CdSe_{0.72}S_{0.28}$/CdS/ZnS nanoplates suspended in water (10 mg/mL) were mixed with 20 mL of water, tetraethoxysilane (80 w%), Zirconium propoxide (20 w%) and ammonia, then loaded on a spray-drying set-up. The liquid was sprayed towards a tube furnace heated at a temperature of 300°C, with a nitrogen flow. The composite particles $CdSe_{0.72}S_{0.28}$/CdS/ZnS in $Si_{0.89}Zr_{0.11}O_2$ matrix were collected at the surface of a filter.
Composite particles (#26) are obtained by a simultaneous spray process: 500 $\mu$L of nanoplates or nanosphere suspended in heptane (10 mg/mL) were mixed with aluminium tri-sec butoxide (70 w%), zirconium propoxide (30 w%) and 20 mL of cyclohexane, then loaded on a spray-drying set-up. On another side, an aqueous solution was prepared and loaded the same spray-drying set-up, but at a different location than the first heptane solution.
The two liquids were sprayed simultaneously towards a tube furnace heated at a temperature of 300°C, with a nitrogen flow. The composite particles comprising nanoplates or nanospheres in a matrix comprising 70 mol% $Al_2O_3$ and 30 mol% $ZrO_2$ were collected at the surface of a filter.
Composite particles (#27) are obtained by a simultaneous spray process: 500 $\mu$L of nanoplates or nanosphere suspended in heptane (10 mg/mL) were mixed with Zirconium propoxide and 20 mL of cyclohexane, then loaded on a spray-drying set-up. On another side, an aqueous solution was prepared and loaded the same spray-drying set-up, but at a different location than the first heptane solution. The two liquids were sprayed simultaneously towards a tube furnace heated at a temperature of 300°C, with a nitrogen flow. The composite particles comprising nanoplates or nanospheres in $ZrO_2$ were collected at the surface of a filter.

Composite particles (#28) are obtained by a simultaneous spray process: 500 $\mu$L of nanoplates or nanosphere suspended in heptane (10 mg/mL) were mixed with Hafnium n-butoxide and 20 mL of cyclohexane, then loaded on a spray-drying set-up. On another side, an aqueous solution was prepared and loaded the same spray-drying set-up, but at a different location than the first heptane solution. The two liquids were sprayed simultaneously towards a tube furnace heated at a temperature of 300°C, with a nitrogen flow. The composite particles comprising nanoplates or nanospheres in $HfO_2$ were collected at the surface of a filter.

Composite particles (#29) are obtained by a simultaneous spray process: 500 $\mu$L of nanoplates or nanosphere suspended in heptane (10 mg/mL) were mixed with Hafnium butoxide (50 w%) and zirconium propoxide (50 w%) and 20 mL of cyclohexane, then loaded on a spray-drying set-up. On another side, an aqueous solution was prepared and loaded the same spray-drying set-up, but at a different location than the first heptane solution. The two liquids were sprayed simultaneously towards a tube furnace heated at a temperature of 300°C, with a nitrogen flow. The composite particles comprising nanoplates or nanospheres in $Hf_{0.4}Zr_{0.6}O_2$ were collected at the surface of a filter.

Functionalization with streptavidin was made differently for nanoparticles not included in a matrix (used in control experiments) and for composite particles.

For nanoparticles not included in a matrix (#3): CdSe/$Cd_{0.3}Zn_{0.7}$S/ZnS nanoplates where complexed with a copolymer comprising 30 mol% of (5, 7-dimercapto)-N-(3-methacrylamidopropyl)heptanamide, 40% of 3-[*N,N,N*-(3-methacrylamidopropyl)-dimethyl-ammonio]propane-1-sulfonate and 30 mol% of methacrylic acid so as to provide a dispersing agent for the nanoparticles. The nanoparticles were then suspended in 210 $\mu$L of a buffer solution at pH 6.0 (50 mmol/L of 2-[4-(2-hydroxyethyl)piperazin-1-yl]ethanesulfonic acid and 100 mmol/L NaCl). 200 $\mu$L of this suspension were then added to 80 $\mu$L of streptavidine-maleimide complex solution (C = 9.47 $10^{-5}$ mol/L in buffer at pH 6.0 - 50 mmol/L of 2-[4-(2-hydroxyethyl)piperazin-1-yl]ethanesulfonic acid and 100 mmol/L NaCl). The resulting mixture was gently stirred for 2 hours at room temperature. Purification was performed on Vivaspin 100 kDa thus yielding nanoparticles functionalized with streptavidin.

For composite particles, a dispersion of composite particles in ultrapure water (500 $\mu$L of a 2 mg/mL stock) was mixed with approximately 1 $\mu$L of a 0.5 mol/L NaOH solution (to ensure a pH between 9 and 10) and 10 $\mu$L of streptavidin solution (6 mg/mL in ultrapure water). After stirring for 90 minutes at room temperature, 100 $\mu$L of BSA solution (0.1 g/mL in ultrapure water) was added and the resulting mixture was again stirred for 1 hour at room temperature. After purification, functionalized composite particles are suspended in 200 $\mu$L of following solution: borate buffer (pH = 10) containing triton X-100 (0.1% v/v), sucrose (10% w/v) and sodium azide (0.05% w/v).

Table 1

| # | Nanoparticle | Matrix | Weight fraction of nanoparticles (%) | Mean size (nm) | $\lambda$ absorption (nm) / FWHM (nm) | $\lambda$ emission (nm) / FWHM (nm) | Migration (Y/N) | Separation (Y/N) | Intensity of light (1) or (2) |
|---|---|---|---|---|---|---|---|---|---|
| 1 | Au NS - 25 nm diameter | None | NA | NA | 525/100 | | Y | Y | |
| 2 | PbS NS - 3 nm diameter | $Al_2O_3$ | 5 | 180 | 895/200 | | Y | Y | |
| 3 | $CdSe/Cd_{0.3}Zn_{0.7}S/ZnS$ NPL $24\times21\times9$ nm | None | NA | NA | | 665/31 | Y | Y | 18 @50 ms |
| 4 | $CdSe_{0.72}S_{0.28}/CdS/ZnS$ NPL $24\times21\times9$ nm | $SiO_2$ | 5 | 250 | | 653/25 | Y | Y | >255 @ 1 ms |
| 5 | $CdSe_{0.72}S_{0.28}/CdS/ZnS$ NPL $24\times21\times9$ nm | $SiO_2$ | 0.5 | 220 | | 653/25 | Y | Y | |
| 6 | $CdSe_{0.72}S_{0.28}/CdS/ZnS$ NPL $24\times21\times9$ nm | $SiO_2$ | 1 | 290 | | 653/25 | Y | Y | |
| 7 | $CdSe_{0.72}S_{0.28}/CdS/ZnS$ NPL $24\times21\times9$ nm | $SiO_2$ | 2 | 250 | | 653/25 | Y | Y | |
| 8 | $CdSe_{0.72}S_{0.28}/CdS/ZnS$ NPL $24\times21\times9$ nm | $SiO_2$ | 12.5 | 230 | | 653/25 | Y | Y | |
| 9 | $CdSe_{0.72}S_{0.28}/CdS/ZnS$ NPL $24\times21\times9$ nm | $SiO_2$ | 25 | 270 | | 653/25 | Y | Y | |
| 10 | $CdSe_{0.72}S_{0.28}/CdS/ZnS$ NPL $24\times21\times9$ nm | $SiO_2$ | 50 | 300 | | 653/25 | Y | Y | |
| 11 | $CdSe_{0.72}S_{0.28}/CdS/ZnS$ NPL $24\times21\times9$ nm | $SiO_2$ | 5 | 740 | | 653/25 | N | | |
| 12 | $CdSe_{0.72}S_{0.28}/CdS/ZnS$ NPL $31\times18\times9$ nm | $Al_2O_3$ | 5 | 160 | | 661/30 | Y | Y | |
| 13 | $CdSe_{0.72}S_{0.28}/CdS/ZnS$ NPL $31\times18\times9$ nm | $Al_2O_3$ | 5 | 810 | | 661/30 | N | | |
| 14 | $CdSe_{0.72}S_{0.28}/CdS/ZnS$ NPL $31\times18\times9$ nm | $Al_2O_3$ | 5 | 460 | | 661/30 | Y | Y | |

| # | Nanoparticle | Matrix | Weight fraction of nanoparticles (%) | Mean size (nm) | λ absorption (nm) / FWHM (nm) | λ emission (nm) / FWHM (nm) | Migration (Y/N) | Separation (Y/N) | Intensity of light (1) or (2) |
|---|---|---|---|---|---|---|---|---|---|
| 15 | CdSe/CdS/ZnS core/shell/shell NS 4.5-1.5-1 nm | $Al_2O_3$ | 5 | 300 | | 620/45 | Y | Y | |
| 16 | $Cd_{0.10}Zn_{0.90}Se_{0.10}S_{0.90}$/ZnS core/shell NS - 4-1 nm | $Al_2O_3$ | 5 | 300 | | 540/37 | Y | Y | |
| 17 | $InP/ZnSe_{0.50}S_{0.50}$/ZnS core/shell/shell NS - 3.5-2-1 nm | $Al_2O_3$ | 5 | 300 | | 630/45 | Y | Y | |
| 18 | InP/ ZnS core /shell NS - 3.5-2 nm | $Al_2O_3$ | 5 | 300 | | 630/40 | Y | Y | |
| 19 | InP/ ZnSe core /shell NS - 3.5-2 nm | $Al_2O_3$ | 5 | 300 | | 635/40 | Y | Y | |
| 20 | $CuInSe_2$/ZnS | $Al_2O_3$ | 5 | 300 | | 625/40 | Y | Y | |
| 21 | $CuInS_2$/ZnS | $Al_2O_3$ | 5 | 300 | | 625/40 | Y | Y | |
| 22 | Carbon dots | $Al_2O_3$ | 5 | 300 | | 625/40 | Y | Y | |
| 23 | Mn doped CdSe/CdS | $Al_2O_3$ | 5 | 300 | | 635/35 | Y | Y | |
| 24 | Ag doped CdSe/CdS | $Al_2O_3$ | 5 | 300 | | 645/35 | Y | Y | |
| 25 | $CdSe_{0.72}S_{0.28}$/CdS/ZnS NPL 24×21×9 nm | $Si_{0.89}Zr_{0.11}O_2$ | 50 | 300 | | 653/25 | Y | Y | |
| 26 | $CdSe_{0.72}S_{0.28}$/CdS/ZnS NPL 24×21×9 nm | 70 w% $Al_2O_3$ and 30 w% $ZrO_2$ | 50 | 300 | | 653/25 | Y | Y | |
| 27 | $CdSe_{0.72}S_{0.28}$/CdS/ZnS NPL 24×21×9 nm | $ZrO_2$ | 50 | 300 | | 653/25 | Y | Y | |
| 28 | $CdSe_{0.72}S_{0.28}$/CdS/ZnS NPL 24x21x9 nm | $HfO_2$ | 50 | 300 | | 653/25 | Y | Y | |
| 29 | $CdSe_{0.72}S_{0.28}$/CdS/ZnS NPL 24×21×9 nm | $Hf_{0.4}Zr_{0.6}O_2$ | 50 | 300 | | 653/25 | Y | Y | |

EP 4 127 716 B1

The present invention is further illustrated by the following examples.

Example 1-a: Device and protocol for assay

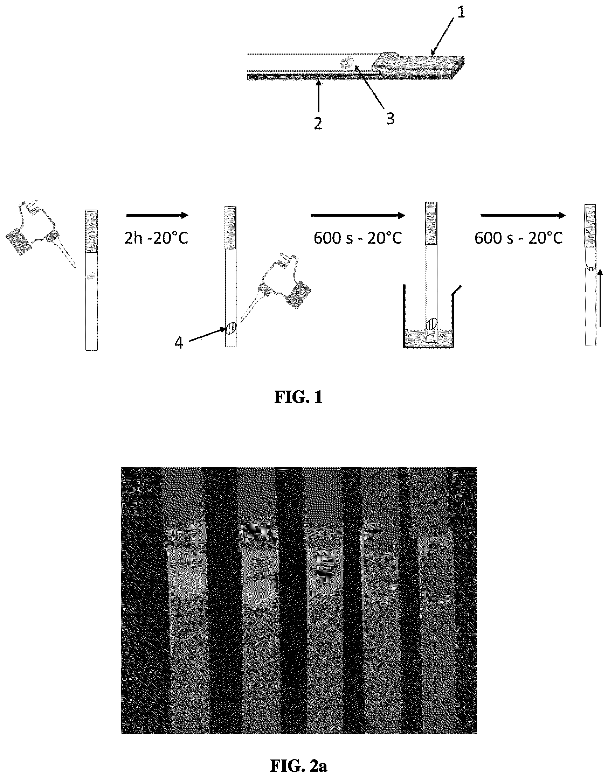

As shown on Figure 1, a pad (1) comprises a nitrocellulose FF80HP porous substrate (2) in the form of a strip (1cm x 7cm). 3 $\mu$L of Bovine Serum Albumin (BSA) - Biotin complex (1 mg/mL, Bovine Serum Albumin, Biotinylated supplied by Thermofisher) is deposited at 1 cm from the higher end of the porous membrane and let for drying 120 minutes at room temperature, thus forming a test zone (3).

Then, a 5 $\mu$L droplet containing composite particles functionalized with streptavidin (borate buffer (pH = 10) containing triton X-100 (0.1% v/v), sucrose (10% w/v) and sodium azide (0.05% w/v) solution) is laid on the lower part of a porous membrane (4). After 600 seconds drying at room temperature, the lower end of the porous membrane is dipped in a solution (borate buffer (pH = 10) containing 1% BSA (w/v), 1% tween 20 (v/v) and triton X-100 (0.1% v/v)) that flows by capillarity along the porous membrane (2) and transport composite particles towards test zone (3). Upon binding of streptavidin with biotin, composite particles attach to the test zone (3). Pad is held vertically. After 600 seconds at room temperature, pad (1) is removed from solution and optical measurements is run on the test zone (3).

In this protocol, the amount of BSA - Biotin complex is the same on each pad and the concentration of composite particles is varied.

Same experiment was run with nanoparticles functionalized with streptavidin, but not included in a matrix.

Samples #1 to #17 were evaluated according to this protocol. Table 1 reports results of migration and separation for all type of composite nanoparticles used. Samples #4 to #11 are disclosed for a better understanding of the invention, but they are not encompassed in the scope of protection of the claims.

In a variant of this protocol, composite particles functionalized with streptavidin are simply mixed with the borate buffer instead of being deposited on the porous membrane and the concentration of BSA - Biotin complex is varied in test zone. Other features of the protocol are unchanged.

Figures 2a-2d shows the result of some of these experiments obtained with the main protocol.

Figure 2a shows fluorescence pictures of pads loaded with CdSe/Cd$_{0.3}$Zn$_{0.7}$S/ZnS nanoplates #3 of dimensions 24x21x9 nm not included in a matrix, with concentration of nanoparticle in the ratio 1, ½, ¼, 1/8 and 1/16 from left to right. Emitted light is red at a wavelength of 653 nm. This experiment is a control showing expected result of migration and separation on nanoparticles. In this experiment, concentration of Bovine Serum Albumin (BSA) - Biotin complex solution deposited on the membrane is 0.2 mg/mL.

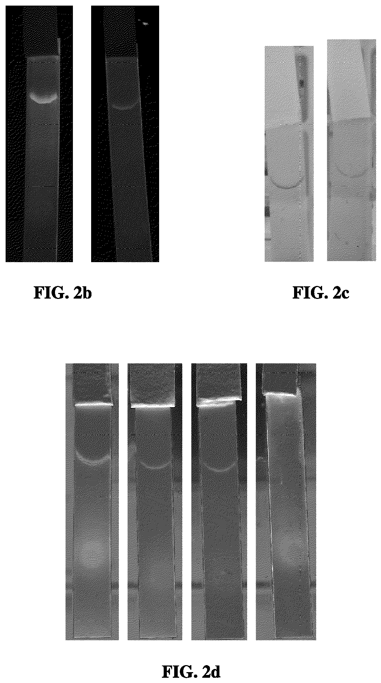

Figure 2b shows fluorescence pictures under UV illumination of pads loaded with composite particles #4, with concentration of composite particles in a ratio 1 (left) and 1/10 (right), for a better understanding of the disclosure, but composite particles #4 are not encompassed in the scope of protection of the claims. Emitted light is red at a wavelength of 653 nm. One can observe bright red lines around the test zone, demonstrating both migration and separation of composite particles.

Figure 2c shows pictures of pads loaded with composite particles #2, with concentration of composite particles in a ratio 1 (left) and 1/4 (right). As nanoparticles here are absorbent, they are identified by a black color on a picture in visible light. One can observe clear black lines around the test zone, demonstrating both migration and separation of composite particles.

Figure 2d shows fluorescence pictures under UV illumination of pads loaded with composite particles #12, with concentration of composite particles in a ratio 1, ¼, 1/8 and 1 from left to right. Emitted light is red at a wavelength of 653 nm. One can observe bright red lines around the test zone, demonstrating both migration and separation of composite particles. Picture on the right is a control: pad does not contain biotin, but only BSA in the test zone. This demonstrates that no fluorescence signal is observed in the test zone. Finally, composite particles are separated by immobilization on biotin in the test zone, as expected.

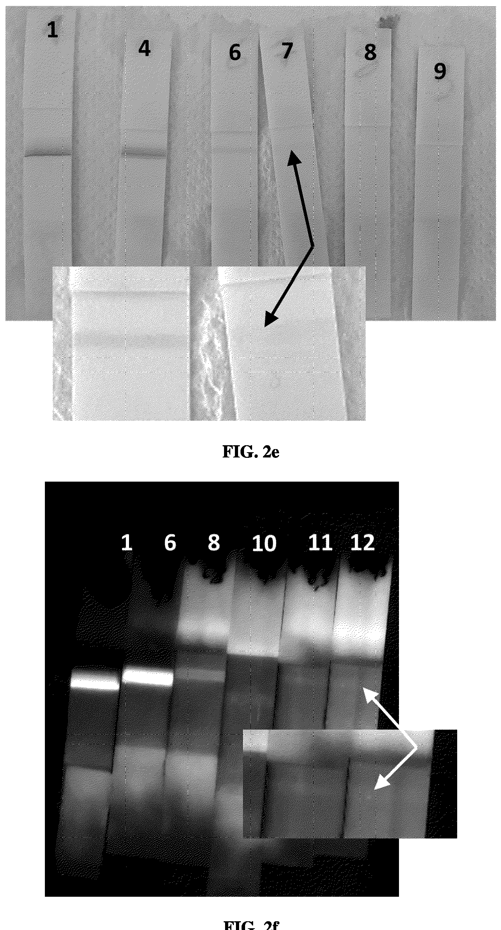

Figures 2e-2g shows the result of some of these experiments obtained with the variant protocol.

A set of 12 pads are prepared with increasing dilution of BSA - Biotin complex: 0.5 mg/ml for pad 1 then twice diluted for each successive pad, yielding BSA - Biotin complex at 5 $\mu$g/ml for pad 7 and 0.16 $\mu$g/ml for pad 12.

A comparative experiment is run with gold nanoparticles not included in a matrix. Figure 2e shows pictures under natural light of pads dipped in a buffer solution comprising 1 $\mu$g/ml gold nanoparticles grafted with streptavidin, diameter 40 nm, supplied by Nanocomposix. A very low signal (pointed by an arrow and evidenced in magnified view) is detected for pad 7: the limit of detection in this protocol is about 5 $\mu$g/ml of BSA - Biotin complex.

An experiment according to present disclosure is run. Figure 2f shows fluorescence pictures under UV illumination of pads dipped in a buffer solution comprising 1 $\mu$g/ml composite nanoparticles #12. A very low signal (pointed by an arrow and evidenced in magnified view) is detected for pad 12: the limit of detection in this protocol is about 0.16 $\mu$g/ml of BSA - Biotin complex, about 30 to 35 times more sensitive than gold nanoparticles used at the same concentration in comparative experiment.

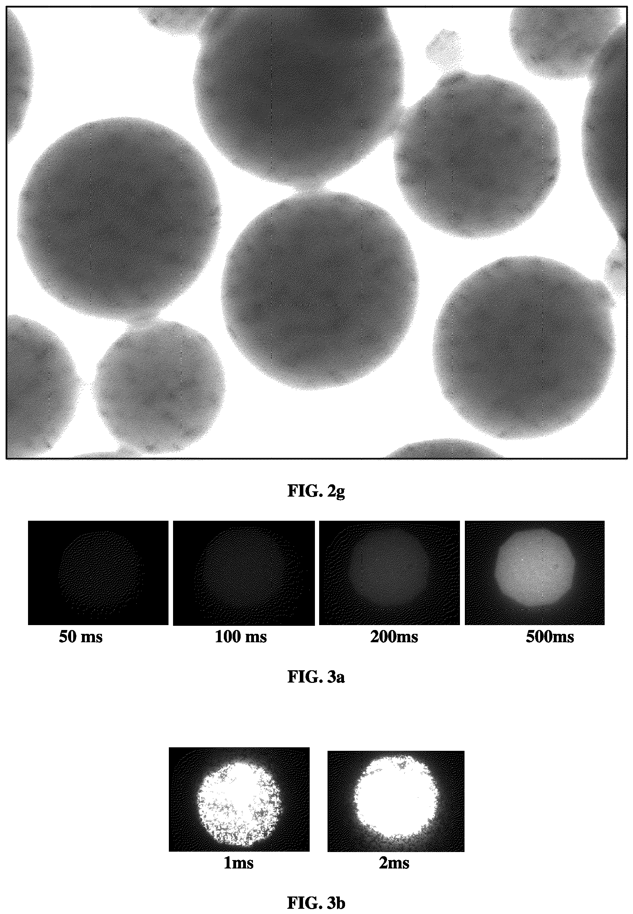

Figure 2g is a TEM picture of composite nanoparticles #12. Average diameter is about 160 nm and one can see darks spots inside composite particles: each spot corresponds to a single nanoplate.

Example 1-b: Comparison of composite particles and nanoparticles alone.

Microscope glass slides (diameter = 13 mm) were first cleaned with a piranha solution (7 mL of 35% hydrogen peroxide solution into 20 mL of 96% concentrated sulfuric acid), then washed, and finally dried for 300 minutes in an oven at 70 °C.

The glass slides were individually placed into small vials and completely coated with 200 μL of Bovine Serum Albumin (BSA) - Biotin complex (1 mg/mL in 0.05 mol/L NaCl solution, Bovine Serum Albumin, Biotinylated supplied by Thermofisher). The coated glass slides were incubated at room temperature for 2 days at 4 °C. The glass slides were then washed with ultrapure water.

Control glass slides were coated with a 200 μL BSA solution (1 mg/mL in 0.05 mol/L NaCl solution) instead of the BSA-biotin solution.

One glass slide was immersed in 100 μL of BSA in a borate buffer solution (pH = 10, BSA concentration: 10 mg/mL). After 10 minutes, 100 μL of the streptavidin functionalized nanoparticles or streptavidin functionalized composite particles suspension was added. After 1h at room temperature, the glass slide was carefully washed with borate buffer (pH = 10) and dried overnight at room temperature. An inverted fluorescence microscope LEICA DMi8, with HXP 120 lamp (Gain = 1, water objective 63x, excitation with a UV-violet band of 20 nm centered at 390 nm), was used to observe the glass slide surface.

In this experiment, functionalized nanoparticles or functionalized composite particles are in large excess as compared to available biotin on the glass slide. One can expect that all biotin sites on glass slides will capture nanoparticles or composite particles. Optical measurements enable to compare sensitivity of test with nanoparticles or composite particles.

Figure 3a shows glass slides image for acquisition times of 50, 100, 200 and 500 ms from left to right for nanoparticles #3, not included in a matrix. Mean intensity measured were respectively 18, 35, 70 and 176 (arbitrary units).

Figure 3b shows glass slides image for acquisition times of 1 ms (left) and 2 ms (right) for composite particles #4 for a better understanding of the disclosure, but composite particles #4 are not encompassed in the scope of protection of the claims. Even at 1 ms, mean intensity could not be measured because of saturation of fluorescence microscope (saturation corresponds to mean intensity of 255 in same arbitrary units).

Comparison of figures 3a and 3b demonstrates that composite particles yield a much more intense optical signal than nanoparticles not included in a matrix, in similar conditions of saturation of sites (biotin) to be detected. Using the linear proportion of integration time, one could evaluate fluorescence intensity of sample #3 about 0.35 @ 1 ms, to be compared with at least 255 @ 1 ms (saturation) for sample #4 in Fig. 3b. Thus, amplification of fluorescence signal obtained using composite particles is about 255/0.35-730, and thus sensitivity of assay would be improved by the same factor 730.

On control glass slide, covered only with BSA, no fluorescence at all was observed.

Table 1 clearly shows that intensity of optical signal is dramatically increased when using composite nanoparticles having a mean size greater than 50 nm and a weight fraction of nanoparticles greater than 0.5%, demonstrating that assay is more sensitive. For the same density of analytes, optical signal measured from a dispersion of large and dense composite particles comprising a given number of nanoparticles is more intense than optical signal measured from a dispersion comprising the same number of isolated nanoparticles or composite particles of small size or small weight fraction.

Example 2

The same pad is used as in example 1-a. However, test zone 3 is prepared by dispensing with a lateral flow reagent dispenser 1 μL/cm of a solution of antibody at 1 mg/mL in a phosphate saline buffer. The antibody is a capture antibody for ovalbumin (AB-Capture-Ova-35).

Besides, composite particles are grafted with another antibody for ovalbumin (AB-Tracker-Ova-1): 10 μg antibody are brought in contact with 100 μg composite particles in a borate 20 mM buffer solution (pH 9). The solution is then incubated during 1 h. After multiple washings, the grafted composite particles are dispersed in borate 10 mM buffer containing 0.1 % Na-Casein (pH 9).

Then, 10 μg grafted composite particles are brought in contact with 100 μl of a solution of Ovalbumin in a buffer (phosphate saline 100 mM and NaCl 150 mM buffer (pH 7.4) containing tween (0.5% v/v) and BSA (0.1% w/v) solution) and let for incubation during 200 s under gentle stirring. Ovalbumin is thus able to complex AB-Tracker-Ova-1 grafted on composite particles.

Last, pad is dipped in the solution of grafted composite particles, as in variant of example 1-a, allowing for migration of composite particles along the pad. When complex of Ovalbumin and grafted composite particles reach test zone 3, ovalbumin is able to form a complex with AB-Capture-Ova-35, thus forming a structure usually known as "sandwich": Ovalbumin acts as a linker between both antibodies and finally, composite particle is anchored to the test zone 3.

After completion of migration (5-10 mn), test zone 3 is illuminated so as to reveal the presence of composite particles by light absorption or light emission.

With this protocol, the sensibility of the assay is determined by varying the concentration of Ovalbumin during incubation. The lowest concentration of Ovalbumin during incubation yielding a detectable optical signal defines the sensibility limit.

Composite particles #12 to #17 have been grafted according to the protocol described above and brought in contact with solutions of Ovalbumin with varying concentrations. Solution 1 has a concentration of 1 ng/ml. Then, solution 1 is diluted twice to obtain next solution (solution 2), until a dilution of 1024 is obtained (10 successive dilutions yielding solution 11).

Figure 5 shows fluorescence pictures under UV illumination of pads after experiment with composite particles #12. Pad number 0 is a comparative experiment, in which incubation is performed without Ovalbumin: no fluorescence is observed. Pad 1 is dipped in solution 1 and shows a very strong fluorescence signal. After dilution of 512 (Ovalbumin concentration of 2 pg/ml - pad 10 dipped in solution 10), fluorescence is still detected - indicated by arrow - whereas after 1024 dilution (pad 11 dipped in solution 11, not shown), no more fluorescence could be detected: sensibility of assay is about 2 pg/ml.

As a comparison, nanoparticles from composite particles #12 were used without alumina encapsulation matrix, as single nanoparticles, with the same protocol. Fluorescence is observed on pad 5, but not on pad 6. Thus, the sensibility of assay is about 50 pg/ml, much lower with single nanoparticles as compared to composite particles, by a factor of about 30.

Example 3

The same pad is used as in example 1-a. However, test zone 3 is prepared by grafting of a capture antibody for ricin (AB-Capture-Rb-37). A solution of antibody at 1 mg/mL in a phosphate saline buffer is printed on test zone 3 using a lateral flow reagent dispenser (1 $\mu$L antibody/cm). In addition, a control zone 4 is prepared by grafting a AffiniPure Goat Anti-Mouse IgG + IgM (H+L). A solution of antibody at 0.5 mg/mL in a phosphate saline buffer is printed on control zone 4 using a lateral flow reagent dispenser (1 $\mu$L antibody/cm).

Besides, composite particles are grafted with another antibody for ricin (AB-Tracker-Rb-35). Antibody Rb35 -1 (5 $\mu$g) are brought in contact with composite particles (100 $\mu$g) in a borate 20 mM buffer solution (pH 9). The solution is then incubated during 1 h. After multiple washings, the grafted composite particles are dispersed in borate 10 mM buffer containing 0.1 % NaCaseine (pH 9).

Then grafted composite particles (10 $\mu$g) are brought in contact with 100 $\mu$l of a solution of ricin in a buffer (phosphate saline 100 mM and NaCl 150 mM buffer (pH 7.4) containing tween (0.5% v/v) and BSA (0.1% w/v) solution) and let for incubation during 200 s. Ricin is thus able to complex AB-Tracker-Rb-35 grafted on composite particles.

Last, pad is dipped in the solution of grafted composite particles, as in variant of example 1-a, allowing for migration of composite particles along the pad. When complex of ricin and grafted composite particles reach test zone 3, ricin is able to form a complex with AB-Capture-Rb-37, thus forming a structure usually known as "sandwich": Ricin acts as a linker between both antibodies and finally, composite particle is anchored to the test zone 3.

After completion of migration (30 mn), test zone 3 is illuminated so as to reveal the presence of composite particles by light absorption or light emission.

With this protocol, the sensibility of the assay is determined by varying the concentration of ricin during incubation. The lowest concentration of ricin during incubation yielding a detectable optical signal defines the sensibility limit.

Composite particles #12 to #29 have been grafted according to the protocol described above and brought in contact with solutions of ricin with varying concentrations. Solution 1' has a concentration of 0.5 ng/ml. Then, solution 1' is diluted twice to obtain next solution (solution 2'), solution 2' is diluted 2.5 times to obtain next solution (solution 3'), solution 3' is diluted twice to obtain next solution (solution 4'), solution 4' is diluted twice to obtain next solution (solution 5'), and solution 5' is diluted twice to obtain next solution (solution 6'), thus a dilution of 40 is obtained with these 5 successive dilutions.

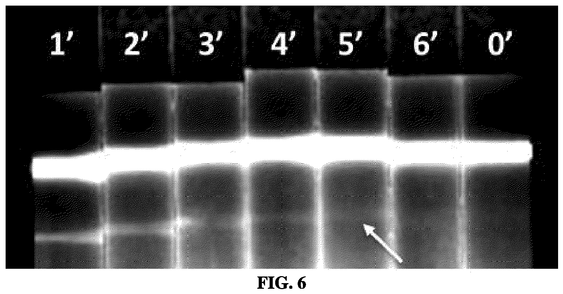

Figure 6 shows fluorescence pictures under UV illumination of pads after experiment with composite particles #12. Pad number 0' is a comparative experiment, in which incubation is performed without Ricin: no fluorescence is observed. Pad 1' shows a very strong fluorescence signal. After dilution of 16 (ricin concentration of 25 pg/ml - pad 5'), fluorescence is still detected - indicated by arrow - whereas after 32 dilutions (pad 6'), no more fluorescence could be detected: sensibility of assay is about 25 pg/ml.

As a comparison, colloidal gold nanoparticles, having a diameter of 40 nm, were used without Alumina encapsulation matrix, as single nanoparticles, in the same protocol. Fluorescence is observed on pad 2', but not on pad 3'. Thus, the sensibility of assay is much lower with single gold nanoparticles as compared to composite particles, by a factor of 10.

**Claims**

1. Composite particle for assay comprising nanoparticles dispersed in a matrix wherein:

   - nanoparticles selectively absorb or selectively emit light;
   - nanoparticles have a size in at least one of its dimensions shorter than 20 nm;
   - weight fraction of nanoparticles in said composite particle is greater than 0.5% and less than 50%;
   - composite particle is functionalized with a specific-binding component; and
   - composite particle has a mean size greater than 50 nm and less than 1000 nm, wherein the matrix is inorganic; wherein the matrix comprises $SiO_2$, $Al_2O_3$, $ZrO_2$, or $HfO_2$, or a mixture thereof, such as $Si_{1-x}Zr_xO_2$, $Al_{2-2x}Zr_{2x}O_{(3+x)}$, or $Hf_{1-x}Z_xrO_2$, x being a rational number between 0 (excluded) and 1 (excluded), and wherein the matrix comprises less than 90% by weight of silica.

2. The composite particle according to claim **1,** wherein nanoparticles are metallic and absorbs selectively light by plasmonic effect.

3. The composite particle according to claim **1,** wherein nanoparticles are inorganic and emit selectively light by luminescence.

4. The composite particle according to any one of claims **1** to **3,** wherein nanoparticles have one dimension shorter than 10 nm, preferably shorter than 5 nm.

5. The composite particle according to any one of claims **1** to **4,** wherein nanoparticles have a shape selected from nanocubes, nanospheres, nanorods, nanowires, nanorings, nanoplates, nanosheets, nanoribbons or nanodisks.

6. The composite particle according to any one of claims **1** to **5,** wherein

   - a first fraction of nanoparticles selectively absorbs or selectively emits light; and
   - a second fraction of nanoparticles selectively absorbs or selectively emits light differently from first fraction of nanoparticles.

7. Method of detection of a target analyte in a sample comprising the steps of:

   i. Providing a sample;
   ii. Letting composite particles according to any one of claims **1** to **6** get in contact with said sample so that the specific-binding component of said composite particles binds with a target analyte;
   iii. Separating said composite particles bound with said target analyte from composite particles not bound with said target analyte; and
   iv. Measuring light absorption or light emission of said composite particles bound with said target analyte or composite particles not bound with said target analyte.

8. The method of detection according to claim **7,** wherein steps ii) and iii) are performed on a strip.

9. The method of detection according to claim **7** or **8,** wherein measure of step iv) is made with a portable device, preferably a mobile phone or a smartphone.

10. An assay test strip, comprising:

   - a porous substrate;
   - a sample receiving zone;
   - a composite particle according to any one of claims 1 to 6 that specifically binds a target analyte; and
   - a detection zone comprising a first immobilized reagent that specifically binds said target analyte and a second immobilized reagent that specifically binds said specific-binding component of said composite particle.

**Patentansprüche**

1. Kompositpartikel für einen Test, das Nanopartikel umfasst, die in einer Matrix dispergiert sind, wobei:

   - die Nanopartikel selektiv Licht absorbieren oder selektiv Licht emittieren;
   - die Nanopartikel in mindestens einer ihrer Abmessungen eine Größe von kürzer als 20 nm aufweisen,
   - die Gewichtsfraktion an Nanopartikeln in dem Kompositpartikel größer als 0,5 % und kleiner als 50 % ist;
   - das Kompositpartikel mit einer spezifisch bindenden Komponente funktionalisiert ist; und
   - das Kompositpartikel eine mittlere Größe von größer als 50 nm und kleiner als 1000 nm aufweist,
   wobei die Matrix anorganisch ist;
   wobei die Matrix $SiO_2$, $Al_2O_3$, $ZrO_2$, oder $HfO_2$, oder ein Gemisch davon umfasst, wie etwa $Si_{1-x}Zr_xO_2$, $Al_{2-2x}Zr_{2x}O_{(3+x)}$, oder $Hf_{1-x}Zr_xO_2$, wobei x eine rationale Zahl zwischen 0 (ausgeschlossen) und 1 (ausgeschlossen) ist, und
   wobei die Matrix weniger als 90 Gewichts-% Siliciumdioxid umfasst.

2. Kompositpartikel nach Anspruch **1,** wobei die Nanopartikel metallisch sind und selektiv Licht durch plasmonischen Effekt absorbieren.

3. Kompositpartikel nach Anspruch **1,** wobei die Nanopartikel anorganisch sind und selektiv Licht durch Lumineszenz emittieren.

4. Kompositpartikel nach einem der Ansprüche **1** bis **3,** wobei die Nanopartikel eine Abmessung aufweisen, die kürzer als 10 nm, bevorzugt kürzer als 5 nm ist.

5. Kompositpartikel nach einem der Ansprüche **1** bis **4,** wobei Nanopartikel eine Form aufweisen, ausgewählt aus Nanowürfeln, Nanokugeln, Nanostäben, Nanodrähten, Nanoringen, Nanoplatten, Nanoblättern, Nanobändern oder Nanoscheiben.

6. Kompositpartikel nach einem der Ansprüche **1** bis **5,** wobei

   - eine erste Fraktion Nanopartikel selektiv Licht absorbiert oder selektiv Licht emittiert; und
   - eine zweite Fraktion Nanopartikel unterschiedlich von der ersten Fraktion Nanopartikel selektiv Licht absorbiert oder selektiv Licht emittiert.

7. Verfahren zum Nachweis eines Zielanalyten in einer Probe, das die folgenden Schritte umfasst:

   i. Bereitstellen einer Probe;
   ii. Inkontaktbringen von Kompositpartikeln nach einem der Ansprüche **1** bis **6** mit der Probe, sodass die spezifisch bindende Komponente der Kompositpartikel mit einem Zielanalyten bindet;
   iii. Trennen der mit dem Zielanalyten gebundenen Kompositpartikel von nicht mit dem Zielanalyten gebundenen Kompositpartikeln; und
   iv. Messen der Lichtabsorption oder Lichtemission der mit dem Zielanalyten gebundenen Kompositpartikel oder nicht mit dem Zielanalyten gebundenen Kompositpartikel.

8. Nachweisverfahren nach Anspruch **7,** wobei die Schritte ii) und iii) auf einem Streifen durchgeführt werden.

9. Nachweisverfahren nach Anspruch **7** oder **8,** wobei die Messung aus Schritt iv) mit einer tragbaren Vorrichtung, bevorzugt einem Mobiltelefon oder einem Smartphone, vorgenommen wird.

10. Teststreifen, umfassend:

    - ein poröses Substrat;
    - eine Probenaufnahmezone;
    - ein Kompositpartikel nach einem der Ansprüche **1** bis **6,** das einen Zielanalyten spezifisch bindet; und
    - eine Nachweiszone, die ein erstes immobilisiertes Reagens, das den Zielanalyten spezifisch bindet, und ein zweites immobilisiertes Reagens umfasst, das die spezifisch bindende Komponente des Kompositpartikels spezifisch bindet.

**Revendications**

1. Particule composite pour un essai comprenant des nanoparticules dispersées dans une matrice dans laquelle :

   - les nanoparticules absorbent sélectivement ou émettent sélectivement de la lumière ;
   - les nanoparticules ont une taille dans au moins une de leurs dimensions inférieure à 20 nm ;
   - la fraction pondérale de nanoparticules dans ladite particule composite est supérieure à 0,5 % et inférieure à 50 % ;
   - la particule composite est fonctionnalisée avec un composant de liaison spécifique ;
   - la particule composite a une taille moyenne supérieure à 50 nm et inférieure à 1 000 nm
   dans laquelle la matrice est inorganique ;
   dans laquelle la matrice comprend du $SiO_2$, $Al_2O_3$, $ZrO_2$, ou $HfO_2$, ou un mélange de ceux-ci, tel que $Si_{1-x}Zr_xO_2$, $Al_{2-2x}Zr_{2x}O_{(3+x)}$, ou $Hf_{1-x}Zr_xO_2$, x étant un nombre rationnel compris entre 0 (exclu) et 1 (exclu), et
   dans laquelle la matrice comprend moins de 90% en poids de silice.

2. Particule composite selon la revendication **1,** dans laquelle les nanoparticules sont métalliques et absorbent sélectivement la lumière par effet plasmonique.

3. Particule composite selon la revendication **1,** dans laquelle les nanoparticules sont inorganiques et émettent sélectivement de la lumière par luminescence.

4. Particule composite selon l'une quelconque des revendications **1** à **3,** dans laquelle les nanoparticules ont une dimension inférieure à 10 nm, de préférence inférieure à 5 nm.

5. Particule composite selon l'une quelconque des revendications **1** à **4,** dans laquelle les nanoparticules ont une forme choisie parmi des nanocubes, des nanosphères, des nanobâtonnets, des nanofils, des nanoanneaux, des nanoplaques, des nanofeuilles, des nanorubans ou des nanodisques.

6. Particule composite selon l'une quelconque des revendications **1** à **5,** dans laquelle

   - une première fraction de nanoparticules absorbe sélectivement ou émet sélectivement de la lumière ; et
   - une seconde fraction de nanoparticules absorbe sélectivement ou émet sélectivement de la lumière différemment de la première fraction de nanoparticules.

7. Procédé de détection d'un analyte cible dans un échantillon comprenant les étapes suivantes :

   i. Fournir un échantillon ;
   ii. Laisser des particules composites selon l'une quelconque des revendications **1** à **6** entrer en contact avec ledit échantillon de sorte que le composant de liaison spécifique desdites particules composites se lie à un analyte cible ;
   iii. Séparer lesdites particules composites liées audit analyte cible des particules composites non liées audit analyte cible ; et
   iv. Mesurer l'absorption lumineuse ou de l'émission lumineuse desdites particules composites liées audit analyte cible ou des particules composites non liées audit analyte cible.

8. Procédé de détection selon la revendication **7,** dans lequel les étapes ii) et iii) sont réalisées sur une bandelette.

9. Procédé de détection selon la revendication **7** ou **8,** dans lequel la mesure de l'étape iv) est réalisée avec un appareil portable, de préférence un téléphone mobile ou un smartphone.

10. Une bandelette de test d'analyse, comprenant :

    - un substrat poreux ;
    - une zone de réception d'échantillon ;
    - une particule composite selon l'une quelconque des revendications **1** à **6** qui se lie spécifiquement à un analyte cible ; et
    - une zone de détection comprenant un premier réactif immobilisé qui se lie spécifiquement audit analyte cible et un second réactif immobilisé qui se lie spécifiquement audit composant de liaison spécifique de ladite particule composite.

**FIG. 1**

**FIG. 2a**

**FIG. 2b**

**FIG. 2c**

**FIG. 2d**

**FIG. 2e**

FIG. 2f

**FIG. 2g**

| 50 ms | 100 ms | 200ms | 500ms |

**FIG. 3a**

| 1ms | 2ms |

**FIG. 3b**

**FIG. 4**

**FIG. 5**

**FIG. 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2428489 A
- US 2016139137 A
- WO 2018220160 A
- US 2009294742 A

### Non-patent literature cited in the description

- **KANAHARA et al.** Fabrication of gold nanoparticle-polymer composite particles with raspberry, core-shell and amorphous morphologies at room temperature via electrostatic interactions and diffusion. *Soft Matter,* 2014, vol. 10 (2), 275-280
- **MATSUMURA et al.** Metal (Au, Pt) nanoparticle-latex nanocomposites as probes for immunochromatographic test strips with enhanced sensitivity. *ACS Applied Materials and Interfaces,* 2018, vol. 10 (38), 31977-31987
- **LI et al.** Ultrasensitive lateral-flow assays based on quantum dot encapsulations with signal amplification. *Journal of nanoparticle research,* 2018, vol. 20 (5), 1-14
- **ZHOU et al.** QDs embedded copolymer nanospheres prepared with a simple self-stable precipitation polymerization method for Lysteria monocytogenes detection. *Optical Materials,* 2019, vol. 99, 1-14
- **ZHANG et al.** Fluorescent microbeads for point-of-care testing: a review. *Mikrochimica Acta,* 2019, vol. 186 (6), 1-21